# FASCICULE DE BREVET EUROPEEN

(11) **EP 4 279 918 B1**
(45) Date de publication et mention de la délivrance du brevet: **23.07.2025**
(21) Numéro de dépôt: 23173069.8
(22) Date de dépôt: 12.05.2023
(51) Int. Cl.: G01N 33/00, G01N 33/24, A01G 25/16

(54) **PROCEDE DE SUIVI DE L'HUMIDITE D'UN SOL, STATION ET INSTALLATION DE MESURE D'HUMIDITE D'UN SOL**
VERFAHREN ZUR ÜBERWACHUNG DER BODENFEUCHTIGKEIT, STATION UND ANLAGE ZUR MESSUNG DER BODENFEUCHTIGKEIT
METHOD FOR MONITORING THE HUMIDITY OF A SOIL, STATION AND INSTALLATION FOR MEASURING THE HUMIDITY OF A SOIL

(30) Priorité: 18.05.2022 FR 2204753
(43) Date de publication de la demande: 22.11.2023
(73) Titulaire: Urbansense, 78000 Versailles (FR)
(72) Inventeur: BUR, Thomas, 78000 Versailles (FR)
(74) Mandataire: IP Trust

(56) Documents cités:
- US-A- 5 601 236
- US-A1- 2018 224 382
- L'ÉQUIPE URBASENSE: "7 Minuten zum Verständnis der agronomischen Bewässerung von Urbasense VOST", 20 August 2020 (2020-08-20), XP093005837, Retrieved from the Internet <URL:https://www.youtube.com/watch?v=2pemCq_TulQ> [retrieved on 20221207]
- VURAN MEHMET C ET AL: "Internet of underground things in precision agriculture: Architecture and technology aspects", AD HOC NETWORKS, ELSEVIER, AMSTERDAM, NL, vol. 81, 27 July 2018 (2018-07-27), pages 160 - 173, XP085523576, ISSN: 1570-8705, DOI: 10.1016/J.ADHOC.2018.07.017

## Description

### Domaine technique

L'invention se place dans le domaine de l'agronomie et concerne un procédé de suivi de l'humidité d'un sol, une station de mesure de l'humidité d'un sol ainsi qu'une installation de suivi de l'humidité d'un sol.

Le terme végétal selon l'invention s'étend à tout type de végétal destiné à être planté au sol en culture pérennes ou annuelles et apte à développer un réseau racinaire, tels que les arbre, les arbustes, les gazons, etc.

### Technique antérieure

Il est admis aujourd'hui que les arbres urbains jouent un rôle écosystémique bénéfique sur l'environnement et sur la santé humaine de part l'élimination des polluants atmosphériques, le stockage et la séquestration du carbone, la protection contre les tempêtes, l'atténuation des impacts des eaux de ruissellement et des inondations, la limitation des îlots de chaleur et même l'amélioration du développement cognitif.

Ils sont donc considérés parmi les meilleures alternatives pour s'adapter au changements météorologiques et climatiques subis dans nos villes.

Malgré ces nombreux bénéfices, la plantation d'arbres en ville est aujourd'hui fortement consommatrice d'eau avec une fort taux d'échec de reprise de l'arbre dans son milieu, impliquant in fine la mort de l'arbre.

En effet, les jeunes arbres nouvellement transplantés ont besoin d'être correctement irrigués durant leur phase de développement racinaire pour leur permettre de s'ancrer durablement dans leur environnement de plantation.

Selon l'espèce et la configuration du lieu de plantation, cet apport peut être prolongé et systématisé pour maintenir en vie les arbres dans un environnement contraint.

Il existe donc aujourd'hui un réel besoin de limiter cette consommation d'eau tout en garantissant une bonne reprise de l'arbre.

Certains procédés utilisent aujourd'hui des données physiologiques et morphologiques des arbres, telles que la croissance des pousses, des feuilles, du tronc ou des tiges pour estimer les besoins en eau de l'arbre.

D'autres procédés se basent sur des expériences menées *in vitro* pour comprendre les phénomènes de la croissance racinaire quand d'autres utilisent des données d'imagerie capables de représenter la structure architecturale et fonctionnelle du développement racinaire.

Il existe également des procédés d'observations *in situ* (excavation, arrachage etc.) qui sont peu écologiques puisqu'ils nécessitent de détruire un grand nombre d'arbres afin d'évaluer un certain développement racinaire dans le temps.

Enfin, il existe la méthode par suivi d'humidité, déjà utilisée en arboriculture pour aider les agriculteurs à économiser l'eau.

Cette méthode, décrite dans le document US6719488, consiste à utiliser des capteurs d'humidité disposés dans le sol pour suivre les besoins en eau des arbres.

Cela permet de mettre en évidence les excès et les manques d'eau dans le sol, de suivre l'état de dessèchement des différents horizons du sol de plantation, et de pouvoir prédire une certaine date et une certaine quantité d'eau à apporter pour réhumecter le sol.

D'autres méthodes telles que celle décrite dans le document US5601236 permet de déterminer l'état de l'humidité du sol à des points précis pour faire de l'arrosage « chirurgical » tandis que la méthode décrite dans le document US2018/224382 cherche à mesurer l'humidité du sol dans le temps pour définir un certain statut des racines.

Ces méthodes ne sont cependant pas suffisamment fiables pour évaluer et optimiser la reprise de l'arbre dans son milieu tout en limitant la consommation d'eau.

### Exposé de l'invention

L'objectif de l'invention est donc de résoudre les problèmes précités.

A cet effet, l'invention porte sur un procédé de suivi de l'humidité d'un sol, caractérisé en ce qu'il comprend les étapes consistant à :
- alimenter automatiquement une base de données numérique en temps réel par des données représentatives de l'humidité du sol mesurées à l'aide de plusieurs capteurs d'humidité disposés dans le sol et répartis sur un chemin estimé de croissance des racines d'un végétal,
- calculer à l'aide d'une unité centrale et au niveau de chaque capteur, une vitesse d'évolution de l'humidité du sol à partir des données d'humidité mesurées, et
- calculer à l'aide de l'unité centrale une somme du nombre de capteurs qui se succèdent sur le chemin estimé de croissance des racines du végétal en partant du capteur disposé au plus près des racines et pour lesquels la vitesse d'évolution de l'humidité du sol est supérieure à une vitesse seuil correspondant à une certaine vitesse de diminution de l'humidité du sol au-dessus de laquelle la diminution de l'humidité du sol est attribuée à la présence de racines, et à créer en retour à l'aide de l'unité centrale une donnée numérique représentative d'un certain volume de répartition des racines dudit végétal correspondant à ladite somme calculée.

Les racines assèchent le sol de façon significative à travers leur activité.

L'idée à la base de l'invention est d'utiliser la cinétique d'évolution de l'humidité du sol au niveau de capteurs disposés dans le sol sur un chemin supposé de développement des racines, pour déterminer en retour un certain volume de répartition des racines du végétal.

Le volume de répartition des racines permet d'avoir une meilleure idée en temps réel de la reprise racinaire du végétal dans son milieu, c'est-à-dire à un déploiement des racines du végétal dans le sol pour chercher de l'eau et ancrer le végétal dans le sol.

Le procédé de mesure de l'activité racinaire du végétal selon l'invention peut également présenter les étapes suivantes consistant à :
- comparer en temps réel à l'aide de l'unité centrale les données d'humidité de chaque capteur pour lesquels la vitesse d'évolution de l'humidité du sol est supérieure à la vitesse seuil, à un seuil d'humidité en dessous duquel la biodisponibilité de l'eau dans le sol n'est pas suffisante pour permettre la croissance des racines du végétal, et en ce que l'unité centrale est paramétrée pour, si l'humidité mesurée au niveau dudit capteur est supérieure au seuil d'humidité, créer une donnée représentative de présence d'une certaine biodisponibilité de l'eau dans le sol suffisante pour permettre la croissance des racines du végétal ;
- identifier une donnée de vitesse d'évolution maximale de l'humidité du sol à partir des données de vitesse d'évolution de l'humidité du sol calculées à partir de l'ensemble des capteurs sur une période donnée, et créer en retour une donnée représentative d'une tendance d'assèchement correspondant à la vitesse d'évolution maximale de l'humidité du sol ;

- mesurer le temps nécessaire pour que la donnée représentative d'une humidité du sol au niveau de chaque capteur diminue selon la vitesse d'évolution de l'humidité du sol maximale jusqu'au seuil d'humidité, et à créer en retour à l'aide de l'unité centrale une donnée de prévision d'assèchement de la réserve utile correspondant au temps mesuré ;
- calculer à l'aide de l'unité centrale et au niveau de chaque capteur un ratio comprenant un numérateur, ledit numérateur étant calculé à partir d'un écart entre les données d'humidité avant réhumectation et les données d'humidité après réhumectation, sur un dénominateur comprenant les données d'humidité avant réhumectation, et à calculer à l'aide de l'unité centrale la moyenne de l'ensemble des ratios calculés pour créer en retour une donnée d'efficacité de réhumectation correspondante ;
- disposer les capteurs d'humidité dans le sol de sorte qu'un premier capteur soit au plus près d'une racine du végétal, un second capteur soit éloigné du premier capteur sur un plan horizontal et un troisième capteur soit éloignée du second capteur sur un plan vertical de sorte que le troisième capteur soit plus éloigné du premier capteur que le second capteur.

L'invention s'étend également à une station de suivi de l'humidité du sol comprenant plusieurs capteurs d'humidité, une carte d'acquisition conçue pour recevoir des données numérique d'humidité des capteurs d'humidité, une batterie conçue pour alimenter ladite carte d'acquisition et les capteurs d'humidité, ladite station comprenant un regard de protection conçu pour loger ladite carte d'acquisition et ladite batterie et être enterré à proximité des racines du végétal et la carte d'acquisition est reliée à une antenne de communication radio conçue pour être déportée à l'extérieure du regard de protection jusqu'à la surface du sol.

L'invention s'étend également à une installation de suivi de l'humidité du sol comprenant une station de suivi selon l'invention, une base de données et une unité centrale conçues pour mettre en œuvre le procédé selon l'invention.

L'installation de suivi selon l'invention est particulièrement bien adaptée pour réaliser des mesures d'humidité dans le sol sur le chemin supposé de développement des racines tout en étant capable de communiquer en temps réel les données d'humidité mesurées à une base de données localisée à distance (hors sol).

### Brève description des dessins

La présente invention sera mieux comprise et d'autres avantages apparaîtront à la lecture de la description détaillée du mode de réalisation pris à titre d'exemple nullement limitatif et illustré par les dessins annexés, dans lesquels :
[Fig 1]- la figure 1 est un diagramme représentant les différentes étapes du procédé selon l'invention pour mesurer le volume de répartition des racines ;
[Fig 2]- la figure 2 est une représentation schématique d'une installation de mesure selon l'invention utilisant une station de mesure d'humidité du sol selon l'invention pour mettre en œuvre le procédé de suivi d'humidité du sol selon l'invention.

### Description détaillée de l'invention

L'invention concerne un procédé de suivi de l'humidité du sol 3 pour déterminer le volume de répartition des racines d'un végétal 1.

Le procédé utilise des données d'humidité produites par plusieurs capteurs d'humidité 2 disposés dans le sol 3 fonctionnant en simultané pour faire des mesure de l'humidité du sol dans le temps.

Les capteurs d'humidité 2 selon l'invention sont ici conçus pour mesurer une teneur en eau ou une tension de l'eau.

La conversion de la teneur en eau vers la tension de l'eau et vice et versa étant possible grâce à une fonction pédotransfert adaptée.

De plus les données mesurées de teneur en eau ou de tension de l'eau sont toujours converties en valeur absolue pour faciliter la lecture des données.

A titre d'exemple, lorsque les capteurs d'humidité 2 mesurent une tension de l'eau, les données mesurées sont exprimées en ohm puis converties en mbar bar et enfin converties en valeur absolue.

Les données d'humidité mesurées pour chaque capteur 2 viennent ainsi alimenter automatiquement en temps réel une base de données 5 numérique.

Les données sont enregistrées périodiquement dans la base de données 5, ici par exemple toutes les 8h, puis moyennées.

Les données d'humidité enregistrées sont par exemple comprises entre 0 et 2000 mbar.

Les capteurs d'humidité 2 sont disposés dans le sol 3 sur le chemin de développement des racines du végétal, en partant par exemple de sa motte 4.

En effet, le végétal qui vient d'être transplanté n'a des racines qu'au niveau de sa motte 4, racines qui vont avoir tendances à se développer à l'horizontal puis à la verticale par rapport à la motte.

A titre d'exemple, un premier capteur d'humidité 2a sera disposé au plus près des racines dans la motte 4 du végétal 1, un second capteur d'humidité 2b éloigné du premier capteur d'humidité 2a sur un plan horizontal et un troisième capteur d'humidité 2c éloignée du second capteur d'humidité 2b sur un plan vertical.

A titre d'exemple nullement limitatif, les capteurs d'humidité 2a, 2b, 2c, peuvent être disposées comme suit :
- 2a : dans la motte à une profondeur de 25 cm
- 2b : à 40cm de la bordure de la motte et à une profondeur de 25 cm.
- 2c : à 40cm de la bordure de la motte et à une profondeur de 75 cm.

Aussi, en plus des données d'humidité mesurées par les capteurs d'humidité 2 en temps réel, la base de données 5 selon l'invention comprend, pour chaque végétal mesuré, au moins l'un ou plusieurs des critères distinctifs suivants : le type d'espèce du végétal (par exemple son essence, le genre, etc.), la région climatique dans laquelle se trouve le végétal, la texture de la motte, l'année de plantation, le contexte de plantation (mélange terre pierre, pleine terre ou fosse de plantation) et les coordonnées géographiques du végétal.

La base de données 5 comprend également une vitesse seuil correspondant à une certaine vitesse de diminution de l'humidité du sol au-dessus de laquelle la diminution de l'humidité du sol est attribuée à la présence de racines.

On peut donc considérer qu'une vitesse de diminution de l'humidité du sol en-dessous de la vitesse seuil correspond sensiblement à une vitesse d'assèchement du sol par évaporation.

La vitesse seuil pourra également variée en fonction des critères distinctifs listés ci-avant.

La vitesse seuil a ici été déterminée à l'avance en réalisant une analyse in situ sur un nombre important de végétaux (arbres, arbustes, etc.). Des végétaux plantés simultanément ont été déterrés successivement au cours d'une année pour pouvoir observer l'évolution des racines.

La vitesse seuil selon l'invention sera par exemple comprise entre 0.1 et 10 mbar/heure en fonction du choix des différents critères distinctifs listés ci-avant.

Le procédé selon l'invention requière également l'utilisation d'une unité centrale 6 paramétrée pour récupérer et traiter les données de la base de données 5.

L'unité centrale 6 selon l'invention est par exemple paramétrée pour créer des données numériques représentatives d'un certain volume de répartition des racines du végétal dans le sol, permettant d'avoir une information sur la présence de racines sur le chemin supposé de développement des racines du végétal.

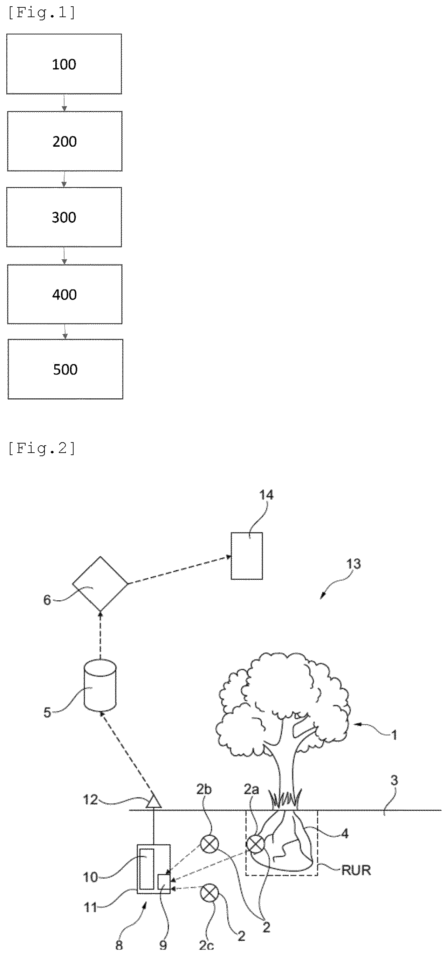

Pour cela, l'unité centrale 6 réalise une succession d'étape illustrées sur la figure 1 et décrites ci-après :
A une étape 100, chaque capteur d'humidité 2 fait des mesures d'humidité et alimente la base de données 5 puis à une étape 200 l'unité centrale 6 calcule une vitesse d'évolution de l'humidité du sol à partir des données d'humidité mesurées pour chaque capteur d'humidité 2.

A une étape 300, l'unité centrale 6 compare la vitesse d'évolution de l'humidité du sol à la vitesse seuil, puis, calcule à une étape 400 à l'aide de l'unité centrale une somme du nombre de capteurs qui se succèdent sur le chemin estimé de croissance des racines du végétal en partant du capteur disposé au plus près des racines et pour lesquels la vitesse d'évolution de l'humidité du sol est supérieure à la vitesse seuil.

A l'étape 500, l'unité centrale 6 créée une donnée numérique représentative d'un certain volume de répartition des racines dudit végétal correspondant à la somme du nombre de capteurs calculée.

A titre d'exemple, lorsque la somme est égale à trois, cela signifie que les trois capteurs d'humidité successifs les plus proches des racines représentent sensiblement le volume de répartition des racines.

L'unité centrale pourra également être paramétrée pour déterminer, à partir de données de distance entre chacun des capteurs d'humidité disposés dans le sol mesurées au moment de l'installation des capteur d'humidité, une longueur approximative des racines dans le sol.

Pour cela, l'unité centrale récupère les données de volume de répartition des capteurs et ajoute les distances entre chacun des capteurs concernés pour sortir une valeur de distance correspondant à la longueur estimées des racines.

Sans restreindre la portée de l'invention, l'unité centrale 6 peut également être paramétrée pour déterminer à partir des données d'humidité mesurées, si le végétal dispose d'une certaine réserve utile pour ses racines.

Par réserve utile on entend une biodisponibilité de l'eau dans le sol suffisante pour permettre la croissance du végétal.

Cette biodisponibilité est limitée par un seuil d'humidité critique en dessous duquel la biodisponibilité de l'eau dans le sol n'est pas suffisante pour permettre la croissance des racines du végétal. Ce seuil a été déterminé à l'avance, en même temps que la vitesse seuil, c'est-à-dire lors des analyses in situ réalisées sur les végétaux.

Pour déterminer la présence ou l'absence de réserve utile, l'unité centrale est paramétrée pour comparer en temps réel les données d'humidité de chaque capteur, pour lesquels la vitesse d'évolution de l'humidité du sol est supérieure à la vitesse seuil, au seuil d'humidité.

L'unité centrale est ainsi apte à, si l'humidité mesurée au niveau dudit capteur est supérieure au seuil d'humidité, créer une donnée représentative de présence d'une certaine biodisponibilité de l'eau dans le sol suffisante pour permettre la croissance des racines du végétal.

A contrario, en cas de détection d'une absence de biodisponibilité de l'eau dans le sol, l'unité centrale pourra envoyer une donnée représentative d'un besoin en arrosage du végétal.

L'unité centrale pourra également être paramétrée pour déterminer une tendance à l'assèchement du sol au niveau des racines du végétal.

Par tendance à l'assèchement du sol on entend une vitesse d'évolution maximale de l'humidité du sol parmi l'ensemble des vitesses d'évolution de l'humidité du sol calculées.

Pour cela, l'unité centrale est paramétrée pour identifier une donnée de vitesse d'évolution maximale de l'humidité du sol à partir des données de vitesse d'évolution de l'humidité du sol calculées à partir de l'ensemble des capteurs sur une période donnée, et créer en retour une donnée représentative d'une tendance d'assèchement correspondant à la vitesse d'évolution maximale de l'humidité du sol.

La période donnée est par exemple comprise entre 2 semaines et deux mois, de préférence un mois.

Sans restreindre la portée de l'invention, le procédé selon l'invention pourra également prévoir l'assèchement de la réserve utile.

Pour cela, l'unité centrale sera paramétrée pour mesurer le temps nécessaire pour que la donnée représentative d'une humidité du sol au niveau de chaque capteur diminue selon la vitesse d'évolution de l'humidité du sol maximale jusqu'au seuil d'humidité, et à créer en retour à l'aide de l'unité centrale une donnée de prévision d'assèchement de la réserve utile correspondant au temps mesuré.

Enfin, le procédé selon l'invention pourra également déterminer une certaine efficacité de réhumectation lorsque le végétal vient d'être réhumecté par la pluie ou un arrosage volontaire.

Pour cela, l'unité centrale sera paramétrée pour calculer au niveau de chaque capteur un ratio comprenant un numérateur, ledit numérateur étant calculé à partir d'un écart entre les données d'humidité avant réhumectation et les données d'humidité après réhumectation, sur un dénominateur comprenant les données d'humidité avant réhumectation, et à calculer à l'aide de l'unité centrale la moyenne de l'ensemble des ratios calculés pour créer en retour une donnée d'efficacité de réhumectation correspondante.

L'invention s'étend également une station 8 de mesure conçue pour mesurer d'humidité du sol 3.

La station 8 comprend plusieurs capteur d'humidité 2, ici à titre d'exemple nullement limitatif, trois capteurs d'humidité 2a, 2b, 2c représentés sur la figure 2, une carte d'acquisition 9 conçue pour recevoir des données d'humidité dudit au moins un capteur d'humidité 2 et une batterie 10 conçue pour alimenter la carte d'acquisition 9 et ledit capteur d'humidité 2.

La station 8 comprend également un regard de protection 11 conçu pour loger la carte d'acquisition 9 et la batterie 10 et pour être enterrée à proximité de la motte 4 du végétal 1.

Le regard 11 est conçu pour être ouvert par le dessus afin de faciliter l'accès à la batterie 10.

La carte d'acquisition 9 est ici reliée à une antenne 12 de communication radio conçue pour être déportée à l'extérieure du regard 11 de protection au plus près de la surface du sol 3, voir à la surface du sol.

L'invention concerne enfin une installation 13 pour réaliser un suivi d'humidité du sol comprenant une station 8 selon l'invention, une base de données 5 et une unité centrale 6 apte à mettre en œuvre le procédé selon l'invention.

Les données créées par l'unité centrale 6 peuvent être affichées sur un écran 14 d'affichage pour faciliter l'utilisation des différentes données produites.

## Revendications

1. Procédé de suivi de l'humidité d'un sol comprenant les étapes consistant à :
- alimenter automatiquement une base de données (5) numérique en temps réel par des données représentatives de l'humidité du sol mesurées à l'aide de plusieurs capteurs d'humidité (2) disposés dans le sol et répartis sur un chemin estimé de croissance des racines d'un végétal,
- calculer à l'aide d'une unité centrale (6) et au niveau de chaque capteur, une vitesse d'évolution de l'humidité du sol à partir des données d'humidité mesurées, et
- calculer à l'aide de l'unité centrale une somme du nombre de capteurs qui se succèdent sur le chemin estimé de croissance des racines du végétal en partant du capteur disposé au plus près des racines et pour lesquels la vitesse d'évolution de l'humidité du sol est supérieure à une vitesse seuil correspondant à une certaine vitesse de diminution de l'humidité du sol au-dessus de laquelle la diminution de l'humidité du sol est attribuée à la présence de racines, et à créer en retour à l'aide de l'unité centrale une donnée numérique représentative d'un certain volume de répartition des racines dudit végétal correspondant à ladite somme calculée.

2. Procédé de suivi de l'humidité du sol selon la revendication **1, caractérisé en ce qu'**il comprend l'étape consistant à comparer en temps réel à l'aide de l'unité centrale les données d'humidité de chaque capteur pour lesquels la vitesse d'évolution de l'humidité du sol est supérieure à la vitesse seuil, à un seuil d'humidité en dessous duquel la biodisponibilité de l'eau dans le sol n'est pas suffisante pour permettre la croissance des racines du végétal, et **en ce que** l'unité centrale est paramétrée pour, si l'humidité mesurée au niveau dudit capteur est supérieure au seuil d'humidité, créer une donnée représentative de présence d'une certaine biodisponibilité de l'eau dans le sol suffisante pour permettre la croissance des racines du végétal.

3. Procédé de suivi de l'humidité d'un sol selon l'une quelconque des revendications précédentes, **caractérisé en ce qu'**il comprend l'étape consistant à identifier une donnée de vitesse d'évolution maximale de l'humidité du sol à partir des données de vitesse d'évolution de l'humidité du sol calculées à partir de l'ensemble des capteurs sur une période donnée, et créer en retour une donnée représentative d'une tendance d'assèchement correspondant à la vitesse d'évolution maximale de l'humidité du sol.

4. Procédé de suivi de l'humidité du sol selon la revendication 3, **caractérisé en ce qu'**il comprend l'étape consistant à mesurer le temps nécessaire pour que la donnée représentative d'une humidité du sol au niveau de chaque capteur diminue selon la vitesse d'évolution de l'humidité du sol maximale jusqu'au seuil d'humidité, et à créer en retour à l'aide de l'unité centrale une donnée de prévision d'assèchement de la réserve utile correspondant au temps mesuré.

5. Procédé de suivi de l'humidité d'un sol selon l'une quelconque des revendications précédentes, **caractérisé en ce qu'**il comprend l'étape consistant à calculer à l'aide de l'unité centrale et au niveau de chaque capteur un ratio comprenant un numérateur, ledit numérateur étant calculé à partir d'un écart entre les données d'humidité avant réhumectation et les données d'humidité après réhumectation, sur un dénominateur comprenant les données d'humidité avant réhumectation, et à calculer à l'aide de l'unité centrale la moyenne de l'ensemble des ratios calculés pour créer en retour une donnée d'efficacité de réhumectation correspondante.

6. Procédé de suivi de l'humidité d'un sol selon l'une quelconque des revendications précédentes, **caractérisé en ce qu'**il comprend l'étape consistant disposer les capteurs d'humidité dans le sol de sorte qu'un premier capteur (2a) soit disposé au plus près d'une racine du végétal, un second capteur (2b) soit éloigné du premier capteur sur un plan horizontal et un troisième capteur (2c) soit éloignée du second capteur sur un plan vertical de sorte que le troisième capteur soit plus éloigné du premier capteur que le second capteur.

7. Installation (13) de suivi de l'humidité du sol comprenant une station de suivie de l'humidité du sol comprenant plusieurs capteurs d'humidité (2), une carte d'acquisition (9) conçue pour recevoir des données numérique d'humidité des capteurs d'humidité, une batterie (10) conçue pour alimenter ladite carte d'acquisition et les capteurs d'humidité, ladite station comprenant un regard (11) de protection conçu pour loger ladite carte d'acquisition et ladite batterie et être enterré à proximité des racines d'un végétal, et où la carte d'acquisition est reliée à une antenne (12) de communication radio conçue pour être déportée à l'extérieure du regard de protection jusqu'à la surface du sol et où l'installation comprend une base de données et une unité centrale conçues pour mettre en œuvre le procédé selon l'une quelconque des revendications 1 à 6.

## Patentansprüche

1. Verfahren zur Überwachung der Bodenfeuchtigkeit, umfassend die Schritte:
- automatisches Speisen einer digitalen Datenbank (5) in Echtzeit mit Daten, die die Bodenfeuchtigkeit darstellen, die mithilfe mehrerer Feuchtigkeitssensoren (2) gemessen werden, die im Boden angeordnet und über einen geschätzten Wurzelwachstumspfad einer Pflanze verteilt sind,
- Berechnen einer Entwicklungsgeschwindigkeit der Bodenfeuchtigkeit anhand der gemessenen Feuchtigkeitsdaten mithilfe einer Zentraleinheit (6) und an jedem Sensor, und
- Berechnen mithilfe der Zentraleinheit einer Summe der Anzahl der Sensoren, die auf dem geschätzten Wachstumspfad der Pflanzenwurzeln aufeinanderfolgen, ausgehend von dem Sensor, der am nächsten an den Wurzeln angeordnet ist und bei denen die Entwicklungsgeschwindigkeit der Bodenfeuchtigkeit größer ist als eine Schwellenwertgeschwindigkeit, die einer bestimmten Abnahmegeschwindigkeit der Bodenfeuchtigkeit entspricht, oberhalb derer die Abnahme der Bodenfeuchtigkeit auf das Vorhandensein von Wurzeln zurückgeführt wird, und mithilfe der Zentraleinheit, Erzeugen eines digitalen Datenwerts, der ein bestimmtes Verteilungsvolumen der Wurzeln der Pflanze darstellt, das der berechneten Summe entspricht.

2. Verfahren zur Überwachung der Bodenfeuchtigkeit nach Anspruch 1, **dadurch gekennzeichnet, dass** es den Schritt umfasst, mithilfe der Zentraleinheit in Echtzeit die Feuchtigkeitsdaten jedes Sensors zu vergleichen, bei denen die Entwicklungsgeschwindigkeit der Bodenfeuchtigkeit höher als die Schwellengeschwindigkeit ist, mit einem Feuchtigkeitsschwellenwert, unterhalb dessen die Bioverfügbarkeit des Wassers im Boden nicht ausreichend ist, um das Wachstum der Wurzeln der Pflanze zu ermöglichen, und dass die Zentraleinheit so parametriert ist, dass sie, wenn die an dem Sensor gemessene Feuchtigkeit über dem Feuchtigkeitsschwellenwert liegt, einen Datenwert erzeugt, der das Vorhandensein einer bestimmten Bioverfügbarkeit des Wassers im Boden darstellt, die ausreichend ist, um das Wachstum der Wurzeln der Pflanze zu ermöglichen.

3. Verfahren zur Überwachung der Bodenfeuchtigkeit nach einem der vorstehenden Ansprüche, **dadurch gekennzeichnet, dass es** den Schritt umfasst, einen Datenwert für die maximale Entwicklungsgeschwindigkeit der Bodenfeuchtigkeit aus den Entwicklungsgeschwindigkeitsdaten der Bodenfeuchtigkeit zu ermitteln, die anhand aller Sensoren über einen bestimmten Zeitraum berechnet wurden, und im Gegenzug einen Datenwert zu erzeugen, der einen Austrocknungstrend entsprechend der maximalen Entwicklungsgeschwindigkeit der Bodenfeuchtigkeit darstellt.

4. Verfahren zur Überwachung der Bodenfeuchtigkeit nach Anspruch 3, **dadurch gekennzeichnet, dass** es den Schritt umfasst, die Zeit zu messen, die erforderlich ist, damit der Datenwert, der eine Bodenfeuchtigkeit an jedem Sensor darstellt, entsprechend der maximalen Entwicklungsgeschwindigkeit der Bodenfeuchtigkeit bis zum Feuchtigkeitsschwellenwert abnimmt, und im Gegenzug mithilfe der Zentraleinheit einen der gemessenen Zeit entsprechenden Prognosedatenwert für das Austrocknen der Nutzreserve zu erzeugen.

5. Verfahren zur Überwachung der Bodenfeuchtigkeit nach einem der vorstehenden Ansprüche, **dadurch gekennzeichnet, dass** es den Schritt umfasst, mithilfe der Zentraleinheit und an jedem Sensor ein Verhältnis zu berechnen, umfassend einen Zähler, wobei der Zähler aus einer Abweichung zwischen den Feuchtigkeitsdaten vor der Wiederbefeuchtung und den Feuchtigkeitsdaten nach der Wiederbefeuchtung zu einem Nenner berechnet wird, umfassend die Feuchtigkeitsdaten vor der Wiederbefeuchtung, und mithilfe der Zentraleinheit der Durchschnitt aller berechneten Verhältnisse berechnet wird, um im Gegenzug einen entsprechenden Wiederbefeuchtungseffizienz-Datenwert zu erzeugen.

6. Verfahren zur Überwachung der Bodenfeuchtigkeit nach einem der vorstehenden Ansprüche, **dadurch gekennzeichnet, dass** es den Schritt des Anordnens der Feuchtigkeitssensoren im Boden umfasst, so dass ein erster Sensor (2a) möglichst nahe an einer Wurzel der Pflanze angeordnet ist, ein zweiter Sensor (2b) auf einer horizontalen Ebene vom ersten Sensor entfernt ist und ein dritter Sensor (2c) auf einer vertikalen Ebene vom zweiten Sensor entfernt ist, so dass der dritte Sensor weiter vom ersten Sensor entfernt ist als der zweite Sensor.

7. Anlage (13) zur Überwachung der Bodenfeuchtigkeit, umfassend eine Station zur Überwachung der Bodenfeuchtigkeit, umfassend mehrere Feuchtigkeitssensoren (2), eine Erfassungskarte (9), die zum Empfangen digitaler Feuchtigkeitsdaten von den Feuchtigkeitssensoren eingerichtet ist, eine Batterie (10), die zum Speisen der Erfassungskarte und der Feuchtigkeitssensoren eingerichtet ist, wobei die Station einen Schutzschacht (11) zur Aufnahme der Erfassungskarte und der Batterie umfasst, der in der Nähe der Wurzeln einer Pflanze vergraben wird, und wobei die Erfassungskarte mit einer Funkantenne (12) verbunden ist, die außerhalb des Schutzschachts zur Bodenoberfläche bewegt werden kann, und wobei die Anlage eine Datenbank und eine Zentraleinheit umfasst, die zur Durchführung des Verfahrens nach einem der Ansprüche 1 bis 6 eingerichtet sind.

## Claims

1. Method for monitoring soil moisture, comprising the steps of:
- automatically feeding a digital database (5) in real time with data representative of the soil moisture, which data are measured using a plurality of moisture sensors (2) arranged in the soil and distributed along an estimated growth path of roots of a plant;
- calculating, using a central processing unit (6) and at each sensor, a rate of change in the soil moisture from the measured moisture data; and
- calculating, using the central processing unit, a sum of the number of sensors which follow one another along the estimated growth path of the roots of the plant, starting from the sensor arranged closest to the roots, and for which the rate of change in the soil moisture is greater than a threshold rate corresponding to a certain rate of decrease in the soil moisture above which the decrease in the soil moisture is attributed to the presence of roots, and of creating in return, using the central processing unit, a digital datum representative of a certain distribution volume of the roots of said plant corresponding to said calculated sum.

2. Method for monitoring soil moisture according to claim 1,
**characterized in that** it comprises the step of comparing in real time, using the central processing unit, the moisture data from each sensor for which the rate of change in the soil moisture is greater than the threshold rate, with a moisture threshold below which the bioavailability of water in the soil is not sufficient to allow growth of the roots of the plant, and **in that** the central processing unit is parameterized in order, if the moisture measured at said sensor is greater than the moisture threshold, to create a datum representative of the presence of a certain bioavailability of water in the soil sufficient to allow growth of the roots of the plant.

3. Method for monitoring soil moisture according to either of the preceding claims, **characterized in that** it comprises the step of identifying a datum of maximum rate of change in the soil moisture from the data of rate of change in the soil moisture that were calculated from all the sensors over a given period, and creating in return a datum representative of a drying-out trend corresponding to the maximum rate of change in the soil moisture.

4. Method for monitoring soil moisture according to claim 3,
**characterized in that** it comprises the step of measuring the time required for the datum representative of soil moisture at each sensor to decrease according to the maximum rate of change in the soil moisture up to the moisture threshold, and of creating in return, using the central processing unit, a datum predicting the drying out of the useful reserve corresponding to the measured time.

5. Method for monitoring soil moisture according to any of the preceding claims, **characterized in that** it comprises the step of calculating, using the central processing unit and at each sensor, a ratio comprising a numerator, said numerator being calculated from a difference between the moisture data before rehydration and the moisture data after rehydration, and a denominator comprising the moisture data before rehydration, and of calculating, using the central processing unit, the average of all the calculated ratios so as to create in return a corresponding rehydration efficiency datum.

6. Method for monitoring soil moisture according to any of the preceding claims, **characterized in that** it comprises the step of arranging moisture sensors in the soil so that a first sensor (2a) is arranged closest to a root of the plant, a second sensor (2b) is spaced apart from the first sensor on a horizontal plane, and a third sensor (2c) is spaced apart from the second sensor on a vertical plane so that the third sensor is further away from the first sensor than the second sensor.

7. System (13) for monitoring soil moisture comprising a station for monitoring the soil moisture, the station comprising a plurality of moisture sensors (2), a capture card (9) designed to receive digital moisture data from the moisture sensors, a battery (10) designed to power said capture card and the moisture sensors, said station comprising a protective observation well (11) designed to house said capture card and said battery and to be buried close to the roots of a plant, and wherein the capture card is connected to a radio communication antenna (12) designed to be positioned remotely outside the protective observation well as far as the surface of the soil and wherein the system comprises a database and a central processing unit that are designed to implement the method according to any of claims 1 to 6.
